# EUROPEAN PATENT SPECIFICATION

(11) **EP 3 064 209 B1**
(45) Date of publication and mention of the grant of the patent: **06.11.2019**
(21) Application number: 16157991.7
(22) Date of filing: 01.03.2016
(51) Int. Cl.: A61K 31/7028, A61P 1/16, A61P 3/10, A61K 31/704

(54) **COMPOSITION COMPRISING GINSENOSIDE F2 FOR PREVENTING OR TREATING INSULIN RESISTANCE**
ZUBEREITUNG ENTHALTEND GINSENOSID F2 ZUR PRÄVENTION ODER BEHANDLUNG DER INSULINRESISTENZ
COMPOSITION CONTENANT DU GINSENOSIDE F2 POUR PRÉVENIR OU TRAITER LA RÉSISTANCE À L'INSULINE

(30) Priority: 03.03.2015 KR 20150030031
(43) Date of publication of application: 07.09.2016
(73) Proprietor: Intelligent Synthetic Biology Center, Daejeon 34141 (KR)
(72) Inventor: JEONG, Won II, 34191 Daejeon (KR); KIM, Sun Chang, 34141 Daejeon (KR); JUNG, Ju Yeon, 35397 Daejeon (KR); IM, Wan Taek, 34141 Daejeon (KR)
(74) Representative: Ström & Gulliksson AB

(56) References cited:
- EP-A1- 2 992 933
- LIU CAN ET AL: "Association of GLP-1 secretion with anti-hyperlipidemic effect of ginsenosides in high-fat diet fed rats", METABOLISM, CLINICAL AND EXPERIMENTAL, W.B. SAUNDERS CO., PHILADELPHIA, PA, US, vol. 63, no. 10, 22 June 2014 (2014-06-22) , pages 1342-1351, XP029063229, ISSN: 0026-0495, DOI: 10.1016/J.METABOL.2014.06.015
- GAO YANG ET AL: "Ginsenoside Re reduces insulin resistance through activation of PPAR-[gamma] pathway and inhibition of TNF-[alpha] pr", JOURNAL OF ETHNOPHARMACOLOGY, ELSEVIER IRELAND LTD, IE, vol. 147, no. 2, 30 March 2013 (2013-03-30), pages 509-516, XP028588755, ISSN: 0378-8741, DOI: 10.1016/J.JEP.2013.03.057
- SIRAJ FAYEZA MD ET AL: "Ginsenoside F2 possesses anti-obesity activity via binding with PPAR[gamma] and inhibiting adipocyte differentiation in the 3T3-L1 cell line.", JOURNAL OF ENZYME INHIBITION AND MEDICINAL CHEMISTRY FEB 2015, vol. 30, no. 1, February 2015 (2015-02), pages 9-14, XP009190569, ISSN: 1475-6374
- Ling Yang ET AL: "Metabolism and pharmacokinetics of ginsenosides", Asian Journal of Pharmacodynamics and Pharmacokinetics, 1 January 2006 (2006-01-01), pages 103-120, XP055281267, Retrieved from the Internet: URL:https://www.researchgate.net/publicati on/215696902 [retrieved on 2016-06-16]

## Description

### [Technical Field]

The present invention relates to a pharmaceutical composition, a health functional food, and a feed composition for the prevention or treatment of insulin resistance, containing ginsenoside F2.

### [Background Art]

The liver is a vital organ, which is in charge of various metabolic actions, detoxification, decomposition, synthesis, and secretion, and the detailed functions are as follows. First, the liver has the function of controlling energy metabolism, and thus it can metabolize all the nutrients absorbed from foods into energy-producing materials and supply them to the whole body or store therein. Second, the liver has the functions of synthesizing, storing, and distributing about 2,000 different kinds of enzymes, albumin, serum proteins of coagulation factors, bile acids, fats such as phospholipids and cholesterols. Third, the liver has the function of excreting various metabolites down the bile duct into the duodenum, and also has an immunological function thus playing an important role in maintaining our lives. Finally, the liver has the functions of detoxification and decomposition thus capable of detoxifying pharmaceutical drugs, toxic materials, alcoholic beverages, etc. However, the detoxifying function of the liver can easily damage hepatocytes thus causing drug-induced, toxic, non-alcoholic liver disease.

Meanwhile, it has been reported that ginsenoside F2 has an anticancer effect in breast cancer by inducing apoptosis along with autophagy in breast cancer stem cells (CSCs) (Mai TT et al. 2012; 28; 321 (2):144-53) and also that ginsenoside F2 has an anticancer effect against glioblastoma in xenograft model in SD rats (Shin JY et al. 2012; 36 (1): 86-92). Additionally, it has been known that ginsenoside F2 can be used as a cosmetic composition for improving skin wrinkles, skin whitening, or antiacne effect (Korean Patent Application Publication No. 2014-0013795). However, the therapeutic effect of ginsenoside F2 insulin resistance has not been identified yet.

It has been demonstrated that hyperlipidemia and insulin resistance are attenuated effectively in response to ginseng total saponins treatment (Liu, C et al., Metabolism, Clinical and Experimental, 2014, vol. 63, no. 10, pages 1342-1351).

Under the circumstances, the present inventors have endeavored to develop a method for treating insulin resistance, and as a result, have discovered that ginsenoside F2 that can be extracted from ginseng can alleviate insulin resistance and thus can be used for preventing or treating insulin resistance, thereby completing the present invention.

### [Disclosure]

### [Technical Problem]

An object of the present invention is to provide a pharmaceutical composition for the prevention or treatment of insulin resistance, containing ginsenoside F2.

Another object of the present invention is to provide a health functional food for the prevention or improvement of insulin resistance, containing ginsenoside F2.

Still another object of the present invention is to provide a feed composition for the prevention or improvement of insulin resistance, containing ginsenoside F2.

Also disclosed herein is a method for the prevention or treatment of insulin resistance, including administering the pharmaceutical composition to a subject in need thereof.

### [Technical Solution]

In order to achieve the above objects, in an aspect, the present invention provides a pharmaceutical composition for use in the prevention or treatment of insulin resistance, containing ginsenoside F2.

Ginsenoside F2, being a tetracyclic protopanaxadiol (PPD) represented by the following Formula 1, is a kind of minor saponin with relatively higher absorptivity and efficacy compared to those of major saponins.

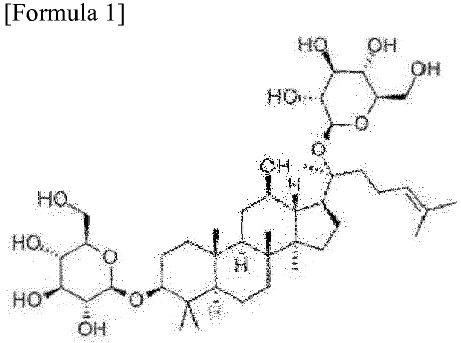

In the present invention, for ginsenoside F2, those commercially available may be purchased and used or it may be isolated from ginseng cultivated in or harvested from the nature or converted from the isolated ginsenoside and used. As an alternative, ginsenoside F2 synthesized by a synthetic method may be used, however, any ginsenoside F2 which shows a therapeutic effect for preventing or treating insulin resistance of the present invention may be used without limitation.

As used herein, the term "insulin resistance" collectively refers to a state in which insulin effects are deteriorated due to reduced sensitivity of the body cells and tissues to respond to insulin, and the composition of the present invention containing ginsenoside F2 can prevent, treat, or improve insulin resistance.

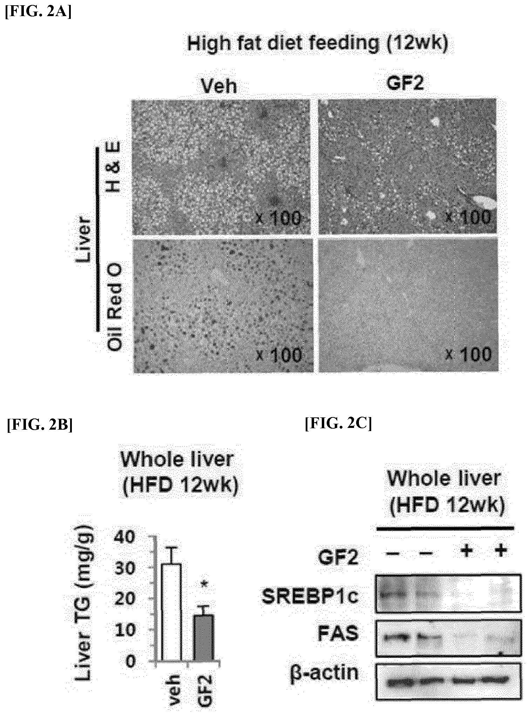

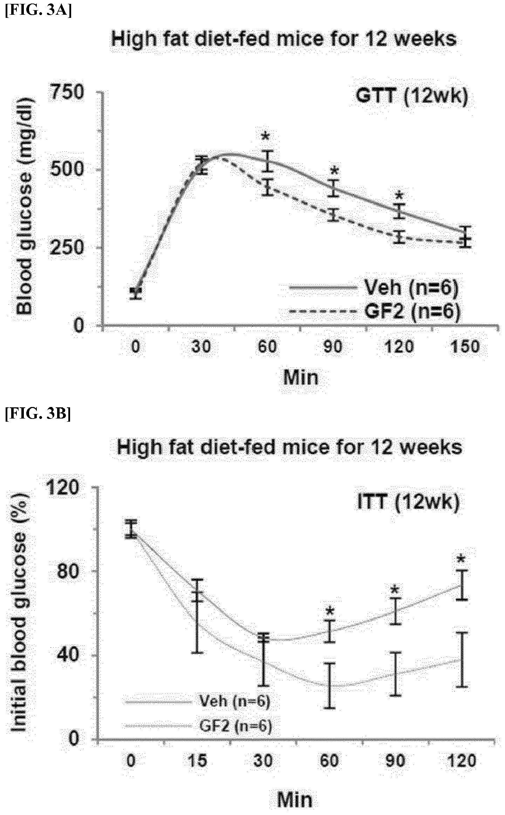

In an experiment, it was confirmed that ginsenoside F2 can inhibit the expressions of SREBP1c and FAS genes for fat synthesis in the liver, which were increased due to high fat diet(HFD), and can also inhibit the fat accumulation in the liver (FIGS. 2A to 2D). Accordingly, it was confirmed that ginsenoside F2 has excellent effect of inhibiting adipogenesis in hepatocytes and hepatic stellate cells, and specifically, that ginsenoside F2 can inhibit the occurrence of nonalcohol-mediated endocannabinoid, which leads to the inhibition of CB1R signaling in the neighboring hepatocytes, thereby preventing adipogenesis and lipid accumulation in the liver. Additionally, it was confirmed that ginsenoside F2 has the effect of improving insulin resistance in a mouse fed with high fat diet (FIGS. 3A and 3B). Accordingly, the pharmaceutical composition of the present invention containing ginsenoside F2 can effectively prevent and treat insulin resistance caused by adipogenesis or lipid accumulation.

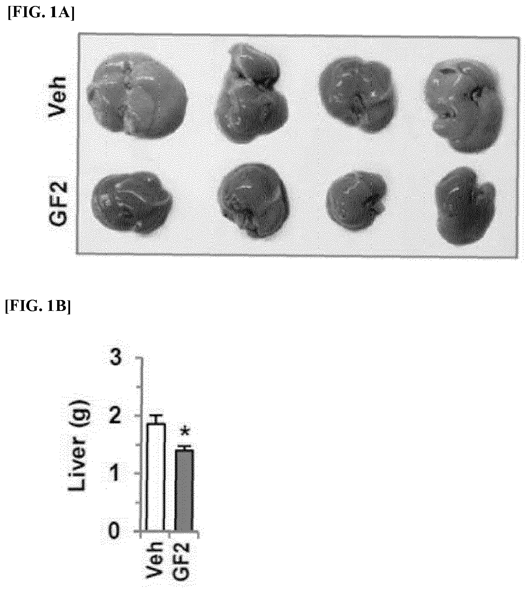

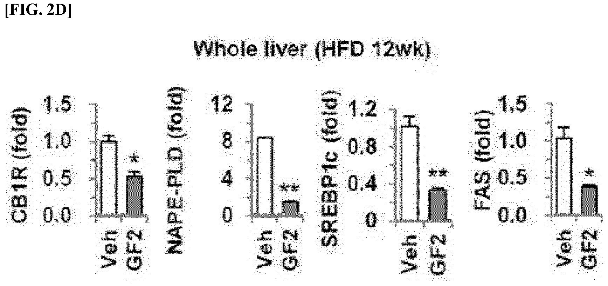

The present inventors have confirmed that the mice fed with high fat diet for 12 weeks showed a substantial decrease in size and weight of their livers (FIGS. 1A and 1B). The decrease of fats was confirmed by H&E staining and Oil Red O staining (FIG. 2).

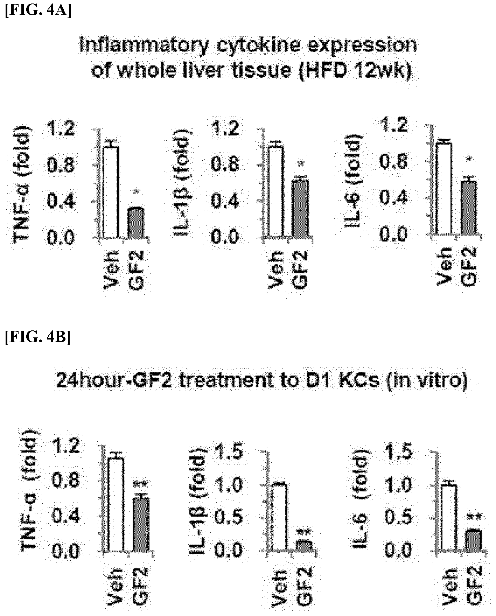

Furthermore, the present inventors have confirmed that the mice fed with ginsenoside F2 have improved insulin sensitivity compared to the mice not fed with ginsenoside F2 (FIG. 3), and that ginsenoside F2 can reduce the expression of inflammatory cytokines, such as TNF-α, IL-1β, and IL-6, in hepatic tissue and hepatocytes of mice fed with high fat diet for a long period of time (FIG. 4).

Accordingly, ginsenoside F2 can improve insulin resistance by reducing the expression of inflammatory cytokines.

In an exemplary embodiment, ginsenoside F2 may be contained in an amount of from 0.01 wt% to 100 wt% based on the total amount of a given pharmaceutical composition, and more preferably from 1 wt% to 80 wt%.

As used herein, the term "prevention" refers to all actions that can inhibit or delay the development of insulin resistance by administering ginsenoside F2 of the present invention to a subject.

As used herein, the term "treatment" may refer to all actions that can improve or beneficially change the symptoms of insulin resistance by administering the composition of the present invention to a subject having insulin resistance.

The pharmaceutical composition of the present invention may be used as a single formulation, or may be prepared in a combined formulation, which additionally includes an approved drug known to have a therapeutic effect for non-alcoholic liver disease, and may be formulated using a pharmaceutically acceptable carrier or excipient to be prepared in a unit dose form or be contained in a multi-dose container.

As used herein, the term "pharmaceutically acceptable carrier" may refer to a carrier or diluent which neither causes significant stimulation to an organism nor abolishes the biological activities or properties of a compound to be administered thereto. A kind of carrier usable in the present invention is not particularly limited, and any carrier may be used as long as it is generally used in the art and is pharmaceutically acceptable. Non-limiting examples of the carrier may include normal saline, sterile water, Ringer's solution, buffered saline, an albumin injection solution, a dextrose solution, a maltodextrin solution, glycerol, ethanol, etc. One or a mixture of two or more thereof may be used. If necessary, additional general additive such as an antioxidant, a buffer, and/or a bacteriostatic agent may be further added and used, and the composition may be formulated into injection formulations such as an aqueous solution, a suspension, an emulsion, or pills, capsules, granules, or tablets by additionally adding a diluent, a dispersant, a surfactant, a binder, a lubricant, etc., and then used.

Further, the pharmaceutical composition of the present invention may include a pharmaceutically effective amount of ginsenoside F2. As used herein, the term "pharmaceutically effective amount" refers to an amount sufficient to treat diseases, at a reasonable benefit/risk ratio applicable to any medical treatment. Generally, the pharmaceutical composition of the present invention may be administered in an amount of 0.001 mg/kg to 1000 mg/kg, preferably 0.05 mg/kg to 200 mg/kg, and more preferably 0.1 mg/kg to 100 mg/kg, singly or divided into several times per day. However, with respect to the objects of the present invention, the specific therapeutically effective dose level for any particular patient may vary depending on various factors such as the type and degree of the response to be achieved, the specific composition, including whether another agent, if any, is employed, the age, body weight, general health conditions, sex and diet of the patient, administration time, administration route, and excretion rate of the composition, duration of treatment, drugs used in combination or concurrently with the specific composition, and similar factors well known in the medical arts.

The pharmaceutical composition of the present invention may be administered alone or in combination with other therapeutic agents, and it may be administered sequentially or simultaneously with conventional therapeutic agents. The composition may be administered in a single or multiple dosage form. It is important to administer the composition in the minimum amount that may exhibit the maximum effect without causing side effects, considering all the factors described above, and this amount may be easily determined by a person skilled in the art.

As used herein, the term "administration" refers to introduction of the pharmaceutical composition of the present invention into a patient using any suitable method. The composition of the present invention may be orally or parenterally administered via any of the common routes, as long as it can reach the target tissue.

The administration method of the pharmaceutical composition according to the present invention is not particularly limited, and the administration may be performed according to the method generally used in the art. As non-limiting examples of the administration, the composition may be administered orally or parenterally. The pharmaceutical composition according to the present invention may be prepared into various formulations according to the desired administration method.

Regarding the administration frequency, the composition of the present invention may be administered once a day or several times a day in divided doses, although not limited thereto.

Furthermore, the present disclosure provides a method of preventing or treating insulin resistance, including administering the composition to a subject suspected of having insulin resistance. For example, the present disclosure provides a method of preventing or treating insulin resistance, including administering the composition to a subject suspected of having insulin resistance, excluding humans.

In particular, the ginsenoside F2, insulin resistance are the same as described above.

As used herein, the term "subject" may refer to all kinds of animals including humans, which already suffer from insulin resistance or at risk for the disease. The animals may be mammals such as cattle, horses, sheep, pigs, goats, camels, antelopes, dogs, and cats, which are in need of treatment for similar symptoms, as well as humans, but are not limited thereto.

Specifically, the prevention or treatment method disclosed herein may include administering a pharmaceutically acceptable amount of the composition to a subject already suffering from insulin resistance or at risk for the disease.

In still another aspect, the present invention provides a health functional food for preventing or improving insulin resistance containing ginsenoside F2.

In particular, ginsenoside F2 insulin resistance are the same as described above.

The ginsenoside F2 is a natural substance which can be extracted from ginseng, white ginseng, wild ginseng, etc. The safety of ginseng has been proven by the long-period of its use. Therefore, it may be eaten raw or prepared in a food which is intended to be used for preventing or improving insulin resistance.

The term "health functional food" is the same term as food for special health use (FOSHU) and refers to a food having high medicinal and medical effects, which is processed to effectively exert a body-regulating function in addition to nutrient supply. The food may be prepared in various forms such as tablets, capsules, powders, granules, liquid, pills, etc., so as to provide a useful effect of preventing or improving insulin resistance.

In particular, the content of the extract included in the food may be in the amount of from 0.01 wt% to 100 wt%, although not particularly limited thereto, and more preferably 1 wt% to 80 wt%, based on the total weight of the food composition.

The food composition of the present invention may further include a sitologically (in the field of the food) acceptable carrier.

A kind of the food, to which the composition containing ginsenoside F2 of the present invention may be added, is not particularly limited, and examples thereof may include various beverages, gums, teas, vitamin complexes, health supplement foods, etc. The food composition may further include other components which do not interfere with the effect of preventing or improving insulin resistance, and a kind thereof is not particularly limited. For example, the food composition, as is most foods, may include various herbal extracts, sitologically acceptable food auxiliary additives, or natural carbohydrates as additional components.

The food supplementary additives may be added during the preparation of health functional foods in various formulations, and may be appropriately determined by one of ordinary skill in the art. Examples thereof may include various nutrients, vitamins, minerals (electrolytes), synthetic and/or natural flavoring agents, colorants and fillers, pectic acid or salts thereof, alginic acid or salts thereof, organic acids, protective colloidal thickening agents, pH modifiers, stabilizers, preservatives, glycerin, alcohols, carbonating agents used in carbonated beverages, etc., but the kind is not limited to these examples.

In particular, the amount of ginsenoside F2 included in the food may be from 0.01 wt% to 100 wt%, and more preferably from 1 wt% to 80 wt%, based on the total weight of the food composition, although not particularly limited thereto.

When the food is a drink, ginsenoside F2 may be included in an amount of 1 g to 30 g, preferably 3 g to 20 g, based on 100 mL of the drink. Additionally, the composition may further include an additive which is commonly used in food compositions to enhance smell, taste, sight, etc. For example, the composition may include vitamins A, C, D, E, B1, B2, B6, and B12, niacin, biotin, folate, pantothenic acid, etc. The composition may also include a mineral, such as zinc (Zn), iron (Fe), calcium (Ca), chromium (Cr), magnesium (Mg), manganese (Mn), and copper (Cu). The composition may also include an amino acid, such as lysine, tryptophan, cysteine, and valine. The composition may further include food additives, such as antiseptics (e.g., potassium sorbate, sodium benzoate, salicylic acid, sodium dehydroacetate, etc.), disinfecting agents (e.g., bleaching powder and high-test bleaching powder, sodium hypochlorite, etc.), antioxidants (e.g., butylhydroxyanisole (BHA), butylhydroxytoluene (BHT), etc.), coloring agents (e.g., tar dye, etc.), color fixing agents (e.g., sodium nitrate, sodium nitrite, etc.), bleaching agents (e.g., sodium sulfite), seasoning agents (e.g., MSG, sodium glutamate, etc.), sweeteners (e.g., dulcin, cyclamate, saccharin, sodium etc.), flavoring agents (vanillin, lactones, etc.), blowing agents (alum, potassium D-bitartrate, etc.), fortifying agents, emulsifying agents, thickening agents, coating agents, gum bases, antifoaming agents, solvents, and improving agents. The additives may be selected according to food types, and they may be used in suitable amounts.

The health functional food of the present invention may be prepared by a method generally used in the art, and it may also be prepared by adding raw materials and ingredients which are generally added in the art during the food preparation. Further, unlike other common drugs, the health functional food may be prepared using foods as raw materials, and thus the health functional food has advantages in that it can avoid side effects associated with long-term administration of drugs and that it is very portable.

In still another aspect, the present invention provides a feed composition for preventing or improving insulin resistance, containing ginsenoside F2.

The ginsenoside F2 and insulin resistance are the same as described above.

The feed composition may include a feed additive. The feed additive of the present invention is classified as an auxiliary additive according to Control of Livestock and Fish Feed Act.

As used herein, the term "feed" may refer to any natural or artificial diet, meal, etc., or components of such meal intended or suitable to be eaten, taken in, or digested by animals.

A kind of the feed is not particularly limited and any feed generally used in the art may be used. Non-limiting examples of the feed may include plant-based feeds, such as grains, nuts, food by-products, seaweeds, fibers, drug by-products, fats and oils, starches, meals, and grain by-products; and animal-based feeds such as proteins, inorganic matters, fats and oils, minerals, single cell proteins, zooplanktons, and foods, but are not limited thereto. These may be used alone or in a mixture of two or more thereof.

### [Advantageous Effects of the Invention]

The pharmaceutical composition according to the present invention comprising ginsenoside F2 can inhibit the adipogenesis and lipid accumulation in the liver, improve insulin sensitivity, inhibit the expression of inflammatory cytokines such as TNF-α, IL-1β, and IL-6 in the Kupffer cell, inhibit the expression of endocannabinoid synthase, thus capable of effectively preventing or treating insulin resistance.

### [BRIEF DESCRIPTION OF THE DRAWINGS]

FIGS. 1A and 1B show the results confirming the effect of ginsenoside F2 on alleviating fatty liver of high fat diet-fed mice.
FIGS. 2A to 2D show the results confirming the degree of adipogenesis in the livers of high fat diet-fed mice.
FIGS. 3A and 3B show the results confirming the effect of ginsenoside F2 on improving insulin sensitivity.
FIGS. 4A and 4B show the results confirming the effect of ginsenoside F2 on inhibiting the expression of inflammatory cytokines.
FIG. 5 shows the result confirming the effect of ginsenoside F2 on inhibiting the expression of NAPE-PLD, FAAH, DAGLα, and DAGLβ in hepatic stellate cells.
FIG. 6 shows a chart illustrating the protective mechanism of ginsenoside F2 against fatty liver and insulin resistance.

### [DETAILED DESCRIPTION OF THE INVENTION]

Hereinafter, the present invention will be described in more detail with reference to the following Examples. However, these Examples are for illustrative purposes only, and the invention is not intended to be limited by these Examples.

### Example 1 : Preparation of ginsenoside F2

The leaves and roots of various ginsengs including *Panax ginseng, Panax quinquefolius,* and *Panax japonicum* were extracted at least twice with 20 volumes of 80% ethanol and dried to obtain crude saponins. The crude saponins were again dissolved in water, adsorbed onto an HP-20 resin, and washed with 100% water to remove saccharides. Then, the resultant was subjected to primary washing using 40% ethanol to remove protopanaxatriol ginsenosides, i.e., Re and Rgl. Subsequently, the resultant was subjected to washing with 80% ethanol to elute protopanaxadiol ginsenosides, i.e., Rbl, Rb2, Rc, and Rd, which were then dried. The protopanaxadiol ginsenoside mixture was reacted as a substrate according to the method described in Korean Patent Application Publication No. 2013-0134930 to obtain at least 70% of ginsenoside F2. Thereafter, ginsenoside F2 to be used as a feed was adsorbed onto an ODS resin, and ethanol at an appropriate concentration was continuously flowed thereonto along the concentration-gradient to obtain a ginsenoside F2 fraction with a purity of 95% or higher, which was dried and used.

### Example 2 : Confirmation of the effect of alleviating fatty liver

The effect of ginsenoside F2 on alleviating fatty liver was confirmed using a mouse model fed with high fat diet(HFD). Eight-week-old male mouse (body weight; 25 g to 28 g) were orally fed with high fat diet and ginsenoside F2 (50 mg/kg) five days a week for a period of 12 weeks. As a result, the group fed with ginsenoside F2 showed a significant decrease in the size of liver (FIG. 1A) and the weight of liver (FIG. 1B), compared to that of the control group. From the results, it was confirmed that ginsenoside F2 has the effect of inhibiting fatty liver production in mice even when they were fed with high fat diet.

### Example 3 : Analysis of liver tissues

Hematoxylin and Eosin (H&E) staining was performed for the observation of the changes in the liver tissues, and the triglycerides in hepatocytes were stained via Oil-red O staining, and the results are shown under x100 magnification (FIG. 2A). As a result, it was confirmed again that the lipid accumulation in hepatocytes was reduced.

Additionally, it was confirmed that ginsenoside F2 treatment lowered the triglyceride level in the liver (FIG. 2B) and also reduced the levels of protein expression of SREBP1c and FAS, which are important factors involved in adipogenesis, and expression of CB1R, NAPE-PLD, SREBP1c, and FAS genes (FIGS. 2C and 2D). Accordingly, it was confirmed that ginsenoside F2 can effectively inhibit adipogenesis in the liver.

### Example 4 : Confirmation of improvement in insulin sensitivity

An eleven-week-old mouse was administered with glucose and insulin and the blood glucose level was monitored in a time-dependent manner. As a result, the group administered with ginsenoside F2 showed a decrease in the glucose level with time compared to that of the control group, thus confirming that ginsenoside F2 has the effect of improving insulin sensitivity in a mouse fed with high fat diet.

### Example 5 : Confirmation of inhibition of expression of inflammatory cytokines

In the mouse fed with high fat diet and ginsenoside F2, ginsenoside F2 was shown to inhibit the expression of various inflammatory cytokines (TNF-α, IL-1β, and IL-6), and the same result was observed in the Kupffer cells (FIG. 4).

Accordingly, it was confirmed that ginsenoside F2 can alleviate hepatitis in a mouse fed with high fat diet by inhibiting the expression of inflammatory cytokines in Kupffer cells.

### Example 6 : Confirmation of inhibition of expression of endocannabinoid synthase in hepatic stellate cells

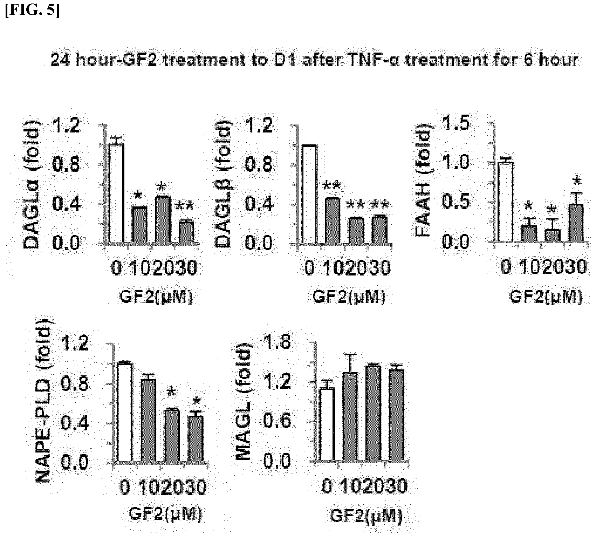

After treating hepatic stellate cells with TNF-α, they were treated with ginsenoside F2 at concentrations of 10 µM, 20 µM, and 30 µM, and cultured for 24 hours. As a result of examining the difference in expression levels in the cultured hepatic stellate cells, it was confirmed that ginsenoside F2 inhibits the expression of DAGLα and DAGLβ, which are enzymes important for the synthesis of 2-AG, a kind of endocannabinoids, and that ginsenoside F2 inhibits the expression of NAPE-PLD and FAAH, which are factors associated with liver damage (FIG. 5).

Conclusively, it was confirmed that ginsenoside F2 inhibits the expression of endocannabinoid synthase in hepatic stellate cells.

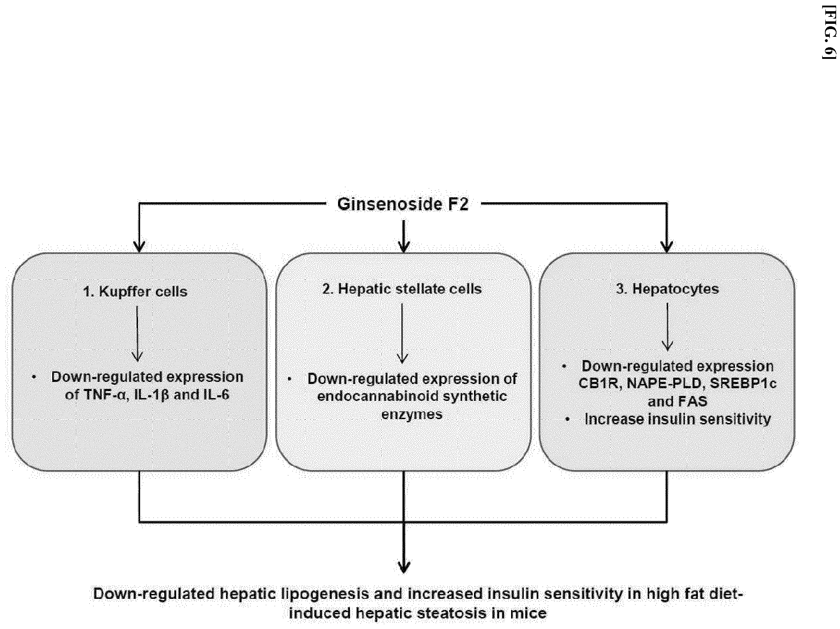

From the foregoing, it was confirmed that ginsenoside F2 inhibits the expression of inflammatory cytokines (TNF-α, IL-1β, and IL-6) in Kupffer cells, whereas ginsenoside F2 inhibits the synthesis of endocannabinoids (DAGLα and DAGLβ) in hepatic stellate cells. Additionally, ginsenoside F2 alleviated liver damage in hepatocytes by inhibiting the expression of genes, such as SREBP1c, FAS, CB1R, and NAPE-PLD, which are associated with adipogenesis. In this regard, FIG. 6 shows a chart illustrating the protective mechanism of ginsenoside F2 against fatty liver and insulin resistance. Accordingly, the composition of the present invention containing ginsenoside F2 can be effectively used for the prevention, treatment, and improvement of insulin resistance.

Those of ordinary skill in the art will recognize that the present invention may be embodied in other specific forms without departing from its spirit or essential characteristics. The described embodiments are to be considered in all respects only as illustrative and not restrictive. The scope of the present invention is, therefore, indicated by the appended claims rather than by the foregoing description. All changes which come within the meaning and range of equivalency of the claims are to be embraced within the scope of the present invention.

## Claims

1. A pharmaceutical composition comprising ginsenoside F2, for use in the prevention or treatment of insulin resistance.

2. A health functional food comprising ginsenoside F2, for use in the prevention or improvement of insulin resistance.

3. The health functional food of claim 2, wherein the health functional food is in the form of powders, granules, tablets, capsules, or beverages.

4. A feed composition comprising ginsenoside F2, for use in the prevention or improvement of insulin resistance.

## Patentansprüche

1. Eine pharmazeutische Zusammensetzung enthaltend Ginsenosid F2, zur 5 Verwendung in der Prävention oder Behandlung von Insulinresistenz.

2. Ein gesundheitliches funktionelles Lebensmittel enthaltend Ginsenosid F2, zur Verwendung in der Prävention oder Verbesserung von Insulinresistenz.

3. Ein gesundheitliches funktionelles Lebensmittel nach Anspruch 2, wobei das gesundheitliche funktionelle Lebensmittel in Form von Pulvern, Granulaten, Tabletten, Kapseln oder Getränken vorliegt.

4. Eine Futterzusammensetzung enthaltend Ginsenosid F2, zur Verwendung in der Prävention oder Verbesserung von Insulinresistenz.

## Revendications

1. Composition pharmaceutique comprenant du ginsenoside F2, pour une utilisation dans la prévention ou le traitement de la résistance à l'insuline.

2. Aliment fonctionnel de santé comprenant du ginsenoside F2, pour une utilisation dans la prévention ou l'amélioration de la résistance à l'insuline.

3. Aliment fonctionnel de santé selon la revendication 2, dans lequel l'aliment fonctionnel de santé se présente sous la forme de poudres, de granules, de comprimés, de capsules, ou de boissons.

4. Composition alimentaire comprenant du ginsenoside F2, pour une utilisation dans la prévention ou l'amélioration de la résistance à l'insuline.
